**Europäisches Patentamt**

**European Patent Office**

**Office européen des brevets**

(19)

(11) Veröffentlichungsnummer: **0 153 680**
**B1**

# (12) EUROPÄISCHE PATENTSCHRIFT

(45) Veröffentlichungstag der Patentschrift:
25.01.89

(21) Anmeldenummer: **85101718.6**

(22) Anmeldetag: **15.02.85**

(51) Int. Cl.⁴: **C 07 D 307/08**

(54) Verfahren zur Herstellung von Tetrahydrofuran.

(30) Priorität: 23.02.84 DE 3406471
05.09.84 DE 3432575

(43) Veröffentlichungstag der Anmeldung:
04.09.85 Patentblatt 85/36

(45) Bekanntmachung des Hinweises auf die Patenterteilung:
25.01.89 Patentblatt 89/4

(84) Benannte Vertragsstaaten:
BE CH DE FR GB IT LI

(56) Entgegenhaltungen:
DE-A-2 303 619
DE-B-2 509 968
DE-C-696 779
DE-C-713 565
DE-C-1 043 342

(73) Patentinhaber: BASF Aktiengesellschaft, Carl-Bosch- Strasse 38, D-6700 Ludwigshafen (DE)

(72) Erfinder: Mueller, Herbert, Dr., Carostrasse 53, D-6710 Frankenthal (DE)
Erfinder: Palm, Christof, Dr., Pariser Strasse 33, D-6700 Ludwigshafen (DE)

## Beschreibung

Die Erfindung betrifft die Herstellung von Tetrahydrofuran durch Dehydratisierung von Butandiol-1,4 unter Verwendung von Phosphorsäure.

Die Dehydratisierung von Butandiol-1,4 zu Tetrahydrofuran hat wegen ihrer großen technischen Bedeutung bereits zu zahlreichen Verfahrensvorschlägen geführt. So wurde bereits vorgeschlagen, die Dehydratisierung in der Flüssigphase oder an fest angeordneten Katalysatoren in der Gasphase durchzuführen, wobei als Dehydratisierungskatalysatoren insbesondere Säuren, wie Schwefelsäure und Phosphorsäure, Kationenaustauscher oder Lewissäuren empfohlen wurden (deutsche Patentschriften 696 779, 711 709, 713 565, 850 750, 10 43 342).

Diese bekannten Verfahren haben jedoch Nachteile, die z. B darin bestehen, daß Nebenreaktionen zu einem nicht tolerierbaren Katalysatorverbrauch führen und im allgemeinen eine 95 %-ige Ausbeute nicht übertroffen wird. Ionenaustauscher haben sich bei den erforderlichen Reaktionstemperaturen als nicht stabil genug erwiesen und sind nur in unzureichendem Maße regenerierbar. Stellt man Tetrahydrofuran nach dem in der DE-PS-1 043 342 beschriebenen Verfahren her, bei dem man Butandiol-1,4 in Gegenwart von 1 bis 5 Gew.% Schwefelsäure bei 100 bis 130°C umsetzt, so müssen die Syntheseapparate aus Korrosionsgründen mit besonders widerstandsfähigem Material, wie Blei ausgekleidet sein. Außerdem sammeln sich in der Schwefelsäure Nebenprodukte an, was dazu führt, daß das Reaktionsgemisch stark zum Schäumen neigt. Man ist deshalb beim Abdestillieren des gebildeten Tetrahydrofurans gezwungen, den entstandenen Sumpfinhalt vorzeitig, d .h. bevor alles Butandiol zu Tetrahydrofuran umgesetzt ist, zu entsorgen. Damit sind zusätzliche Umweltprobleme verbunden. Außerdem hat dieses kontinuierliche Verfahren den Nachteil, daß Schwefelsäure als Katalysator nicht unbegrenzt weiterverwendet werden kann. Obwohl ein Katalysatorverbrauch theoretisch nicht statfindet, treten Verharzungen auf, die zu häufigen Abstellungen und damit zu beträchtlichen Säureverlusten führen. Pro Gewichtsteil Schwefelsäure werden nach der in der DE-PS-1 043 342 beschriebenen Arbeitsweise bis zu 10.000 Teile Butandiol umgesetzt. Bei einer großtechnischen Ausübung des Verfahrens ist also eine erhebliche Menge an Schwefelsäure zu vernichten. Dieser Mangel ist umso gravierender, je unreiner das verwendete Butandiol ist. Undestilliertes Butandiol kann im allgemeinen nicht für die Tetrahydrofuran-Herstellung nach diesem Verfahren verwendet werden, da die angegebene Katalysatorproduktivität nur bei sehr reinem Butandiol gewährleistet ist.

Nun wird Butandiol-1,4 nicht nur zum Zwecke der nachfolgenden Synthese von Tetrahydrofuran hergestellt. Butandiol-1,4 ist vielmehr ein wichtiges Vorprodukt für die Herstellung von Kunststoffen. Bei der für diesen Verwendungszweck erforderlichen Reinigung des Butandiols fällt stets auch eine Butandiol-Fraktion an, die durch Nebenprodukte verunreinigt ist und die für die Herstellung von Tetrahydrofuran gut geeignet wäre. Man müßte dann aber einen höheren Schwefelsäureverbrauch in Kauf nehmen, denn auch diese Verunreinigungen reichern sich vor allem in der Sumpfsäure an. Es kommt zum Schäumen, und das Reaktionsgemisch wird, da sich die Nebenprodukte oder daraus entstandene Polymere in der Schwefelsäure lösen, schnell zähflüssig.

Um diesem Übel abzuhelfen, wird bei dem in der DE-OS-2 303 619 beschriebenen Verfahren ein undestilliertes rohes Butandiol mit Schwefelsäure und mit Tallöl umgesetzt, wobei man die Verunreinigungen mit dem Tallöl kontinuierlich ausschleust. Dadurch erhöht sich zwar die Ausbeute an Tetrahydrofuran, es gehen aber ständig Tallöl und Schwefelsäure verloren.

Bei dem in der DE-PS-711 709 beschriebenen Tetrahydrofuran-Verfahren wird Butandiol-1,4 mit Wasser unter Druck in Gegenwart wasserabspaltender Katalysatoren auf Temperaturen über 250°C erhitzt. Als wasserabspaltende Katalysatoren kommen sowohl heterogene als auch homogene Katalysatoren in Betracht. Obwohl man bei diesem Verfahren verhältnismäßig hohe Ausbeuten erhält, hat es keinen Eingang in die Technik gefunden, weil das Tetrahydrofuran insbesondere durch Dihydrofuran-2,3 und Dihydrofuran-3,4, Carbonylverbindungen und Leichtsieder, wie Butadien verunreinigt ist. Schwerwiegend ist der Gehalt an Dihydrofuranen und Carbonylverbindungen, da diese Verbindungen nicht destillativ aus dem Tetrahydrofuran entfernt werden können. Weitere Verunreinigungen entstehen dadurch, daß bei den hohen Temperaturen gebildetes Tetrahydrofuran in Propylen und Formaldehyd zerfällt.

An die Reinheit von Tetrahydrofuran werden je nach Anwendungzweck mehr oder minder hohe Anforderungen gestellt. Handelsübliches Tetrahydrofuran technischer Qualität besitzt bereits einen sehr hohen Reinheitsgrad. Normalerweise beträgt seine Reinheit über 99,8 %. Selbst ein Tetrahydrofuran mit einem Gehalt an Verunreinigungen von nur 10 bis 50 ppm kann aber für anspruchsvolle Verwendungszwecke, wie für die Herstellung von Polytetrahydrofuran, ungeeignet sein. Ein Tetrahydrofuran mit einer Reinheit, die auch diesen hohen Anforderungen genügt, läßt sich nach dem Verfahren der DE-PS-711 709 nicht herstellen und zwar auch dann nicht, wenn ein noch so hoher destillativer Aufwand getrieben wird. Hier bringen auch die verschiedentlich vorgeschlagenen Reinigungsmethoden für Tetrahydrofuran (US-PS-3 980 672, EP-PS-1 761) nicht den gewünschten Erfolg.

Die in den deutschen Patentschriften 711 709 und 713 565 beschriebenen Verfahren haben sich auch deshalb in der Technik nicht durchsetzen können, weil beim kontinuierlichen Betrieb mit Butandiol-Rohlösungen, wie sie bei der Hydrierung von Butindiol-Rohlösungen anfallen, die durch Umsetzung von Acetylen mit Formaldehyd erhalten werden, schon nach kurzer Zeit Verstopfungen der Rohre durch Harzbildung auftreten. Mit zunehmender Laufzeit verlieren die Reaktoren an Aktivität, obwohl der Zulauf gleich bleibt. Dieser Reaktivitätsminderung kann dadurch entgegengewirkt werden, indem die Rohlösungen nach den Angaben in Annalen der Chemie, 596, Band (1955), Seiten 81 und 82 zuvor mit Kationenaustauschern behandelt werden. Reppe und Mitarbeiter haben an dieser Literaturstelle mitgeteilt, daß die Dehydratisierung des Butandiols zu Tetrahydrofuran eine Gleichgewichtsreaktion ist, die zwar weitgehend auf der Seite des Tetrahydrofurans liegt, aber im Reaktionsprodukt noch soviel Butandiol zuläßt, daß dieses Verfahren nicht wirtschaftlich erscheint. Sie geben hier an, daß ein vollständiger Umsatz von Butandiol-1,4 zu Tetrahydrofuran also nur dann erreicht wird, wenn Tetrahydrofuran im Maße seiner Bildung durch Destillation aus dem Reaktionsgemisch entfernt wird.

Die Aufgabe der Erfindung bestand nun darin, ein Verfahren zur Herstellung von Tetrahydrofuran zu finden, das es ermöglicht, aus rohen wäßrigen Lösungen von Butandiol-1,4 Tetrahydrofuran von so hoher Reinheit herzustellen, daß es für die Herstellung von Polytetrahydrofuran geeignet ist. Es sollten insbesondere auch Umsätze zu Tetrahydrofuran erzielt werden, die außerhalb der oben beschriebenen Gleichgewichtskonzentration, d.h. zur Tetrahydrofuranseite hin verschoben liegen. Das Verfahren sollte außerdem die Bedingung erfüllen, daß es besonders wirtschaftlich und umweltfreundlich ausgeführt werden kann.

Nach dem neuen Verfahren, das diese Aufgaben erfüllt, wird Tetrahydrofuran aus wäßrigen, schwach alkalischen Butandiol-1,4-Lösungen, die durch Umsetzung von Acetylen mit wäßrigem Formaldehyd und katalytische Hydrierung der so hergestellten Butin-2-diol-1,4-Lösung erhalten werden, durch Wasserabspaltung in flüssiger Phase bei höherer Temperatur unter Druck und in Gegenwart einer Säure dadurch hergestellt, daß man zu der rohen wäßrigen Lösung soviel Schwefelsäure gibt, daß die zur Neutralisation der in der Lösung vorhandenen Basenäquivalente erforderlichen Schwefelsäureäquivalente zumindest erreicht oder um bis zu 20 % überschritten werden, die dabei erhaltene Lösung durch Zugabe von Phosphorsäure ansäuert und dann unter Druck auf Temperaturen von 200 bis 260°C erhitzt.

Die wäßrigen schwach alkalischen Lösungen von Butandiol-1,4, die nach dem erfindungsgemäßen Verfahren für die Herstellung von Tetrahydrofuran als Ausgangslösungen herangezogen werden, fallen bei der bekannten und großtechnisch ausgeübten Umsetzung von Acetylen mit wäßrigen Formaldehyd an, bei der wäßrige Lösungen von Butin-2-diol-1,4 entstehen, die der katalytischen Hydrierung unterworfen werden (s. Ullmanns Encyklopädie der techn. Chemie (1953), Band 3, Seiten 109 bis 119, Band 4, Seiten 754 bis 757, DE-AS-2 421 401 und DE-OS-2 536 273.

Die wäßrigen Ausgangslösungen haben z. B. folgende Zusammensetzung: 20 bis 60 Gew.-% Butandiol-1,4, 30 bis 79 Gew.-% Wasser, 1 bis 5 Gew.-% Monoalkohole, wie Methanol, Propanol und Butanol sowie ungesättigte Verbindungen, wie Butendiol. Außerdem können sie in geringen Mengen Carbonylverbindungen, wie Hydroxibutyraldehyd oder die entsprechenden Acetale, polymere Harze sowie anorganische Bestandteile, wie Natriumsalze, gelöste Katalysatorbestandteile und Katalysatorabrieb enthalten. Zu diesen rohen wäßrigen Lösungen von Butandiol-1,4 gibt man zur Neutralisierung Schwefelsäure. Das zur Neutralisation verwendete Schwefelsäureäquivalent darf die Summe der in der Lösung vorhandenen Basenäquivalente nicht unterschreiten. Die Summe der vorhandenen Basenäquivalente kann man durch die Bestimmung der Basenzahl, z. B. durch Titration mit $\frac{n}{10}$ HCl gegen Bromphenolblau, ermitteln. Eine Überschreitung der Schwefelsäureäquivalente um mehr als 20 Äquivalentprozente soll vermieden werden.

Die mit Schwefelsäure neutral gestellte Ausgangslösung wird mit Phosphorsäure angesäuert. Man stellt das wäßrige Gemisch durch die Zugabe der Phosphorsäure auf einen pH-Wert von vorzugsweise 2 bis 3. Die Konzentration der Phosphorsäure in der Butylenglykolausgangslösung liegt im allgemeinen zwischen 0,1 und 0,5 Gew.%. Dann erhitzt man unter autogenem Druck auf Temperaturen von 200 bis 260°C, vorzugsweise 230 bis 250°C. Die Verweilzeiten betragen 2 bis 5 Stunden. Dabei wird das Butandiol selektiv unter Vermeidung der Bildung von Nebenprodukten zu Tetrahydrofuran cyclisiert. Gleichzeitig verbleiben 1 is 2 Gew.% der ursprünglich in der Lösung vorhandenen Butandiol-Menge nicht umgesetzt im Gemisch. Man kann diese geringe Butandiol-Restmenge z. B. dadurch auf 0,1 bis 0,3 Gew.% reduzieren, daß man das so erhitzte flüssige Reaktionsgemisch bei einer mittleren Verweilzeit von mindestens 10 Minuten und in Abwesenheit einer Gasphase auf Temperaturen unter 100°C abkühlt.

Die Umsetzung wird zweckmäßigerweise kontinuierlich durchgeführt, beispielsweise indem man die Rohlösung mit einer Pumpe zunächst durch einen Vorheizer und dann durch ein Verweilzeitrohr preßt, in dem sie den genannten Temperaturen ausgesetzt wird. Der Reaktionsdruck wird mindestens gleich dem Dampfdruck des Umsetzungsproduktes bei Reaktionstemperatur gewählt. Höhere Drücke können gewählt werden, sie beeinflussen aber nicht die Umsetzung.

Die Abkühlung des auf 200 bis 260°C erhitzten flüssigen Reaktionsgemisches nimmt man z. B. in einem Wärmeaustauscher vor, zweckmäßigerweise kontinuierlich in einem Röhrenwärmetauscher. Die mittlere Verweilzeit des Reaktionsgemisches im Wärmeaustauscher, in dem es auf Temperaturen unter 100°C abgekühlt wird, beträgt z. B. 10 bis 45 Minuten, vorzugsweise 15 bis 30 Minuten. Nach dieser Behandlung, die man in dem geschlossenen System und in Abwesenheit einer Gasphase vornimmt, leitet man das Reaktionsgemisch zur Abtrennung des Tetrahydrofurans, zweckmäßigerweise durch Entspannung über ein Druckventil, in eine Destillationskolonne.

Im Sumpfe der Destillierkolonne laufen das ursprünglich im Gemisch enthaltene und das bei der Umsetzung entstandene Wasser fortlaufend ab; während am Kopfe der Destillierkolonne ein bei 64°C siedendes aus ca. 96 Gew-.% Tetrahydrofuran und 4 Gew.-% Wasser bestehendes Azeotrop aus Tetrahydrofuran und Wasser anfällt. Entwässert man dieses Gemisch, so erhält man ein Tetrahydrofuran mit einer Reinheit von über 99,9 Gew.-%. das Produkt läßt sich ohne weitere Reinigungsoperation zur Herstellung von Polytetrahydrofuran verwenden.

Das Verfahren kann absatzweise ausgeführt werden. Vorteilhaft ist natürlich die kontinuierliche Betriebsweise, da in diesem Falle die fühlbare Wärme des Reaktionsaustrages am einfachsten für die destillative Gewinnung des Tetrahydrofuranazeotropes zurückgewonnnen werden kann.

Da durch die beobachtete Abnahme der Restmenge an Butandiol beim Abkühlen des Reaktionsgemisches der Gehalt an Tetrahydrofuran steigt, erhält man eine weitgehend vollständige Umsetzung. Dies ist insofern überraschend, weil man so geringe Butandiol-Restgehalte sonst nur erreicht, wenn man das Ausgangsgemisch beim Erhitzen auf z. B. 250°C langen Verweilzeiten, wie solchen von 6 bis 8 Stunden aussetzt. Daß man das vorteilhafte Ergebnis schon bei einer mittleren Verweilzeit von 2 Stunden erhält, wenn man das Reaktionsgemisch nach dem Erhitzen erfindungsgemäß abkühlt, war nicht vorauszusehen, da die Reaktionsgeschwindigkeit im allgemeinen durch Temperatursteigerungen erhöht wird. Es ist ebenfalls überraschend, daß man z. B. ein schlechteres Ergebnis erhält, wenn man nicht auf Temperaturen unter 100°C, sondern auf Temperaturen unter 150°C abkühlt. In diesem Falle werden nämlich nicht 99,7 bis 99,9, sondern nur 99,7 bis 99,4 Gew.-% des Butandiols in Tetrahydrofuran überführt. Kühlt man schneller als beim erfindungsgemäßen Verfahren vorgesehen ab, so werden die guten Resultate ebenfalls nicht erzielt.

Daß man nach dem Verfahren der Erfindung Tetrahydrofuran aus rohen wäßrigen Lösungen von Butandiol-1,4 in 100 %-iger Ausbeute herstellen kann, muß auch aufgrund der Angaben von Reppe (Annalen der Chemie, 596. Band (1955), Seiten 81 und 82) als überraschend bezeichnet werden. Obwohl beim erfindungsgemäßen Verfahren im stark sauren Bereich und bei hohen Temperaturen gearbeitet wird, hat es sich überraschenderweise gezeigt, daß man als Reaktionsapparate auch solche aus erheblich billigeren Werkstoffen, wie Edelstahl Nr. 1.4571 und Nr. 1.4439 verwenden kann. Daß sich diese Werkstoffe als unter den Verfahrensbedingungen korrosionsbeständig erwiesen haben, konnte nicht erwartet werden.

**Beispiel 1**

Als Ausgangslösung wurde eine rohe wäßrige 50 gew.%-ige Butandiol-1,4-Lösung verwendet, die durch Umsetzung von Acetylen mit wäßrigem Formaldehyd (s. DE-AS-2 421 407) und katalytische Hydrierung der dabei erhaltenen Butin-2-diol-1,4-Lösung (s. DE-OS-2 536 273) hergestellt worden war. Durch Titration mit $\frac{1}{10}$ HCl gegen den Indikator Bromphenolblau wurde eine Basenzahl von 1,0 mg KOH/g in der Lösung festgestellt. Durch Zumischen von 0,1 Gew.-% Schwefelsäure wurde die Lösung auf einen pH-Wert von 7 eingestellt. Danach wurde durch Zugabe von 0,15 Gew.-% Phosphorsäure ein pH-Wert von 2,5 eingestellt.

Die Herstellung des Tetrahydrofurans aus der Butandiol-Lösung wurde in einer Reaktionskaskade (aus Edelstahl 1.4571) durchgeführt, die aus einem Elektrovorheizer und 2 hintereinandergeschalteten Rohrreaktoren mit jeweils 100 Volumenteilen Reaktionsraum bestand. Die Länge der Reaktionsrohre verhielt sich zum Rohrdurchmesser wie 70 : 1.

Die angesäuerte Butandiol-Lösung wurde kontinuierlich durch den Elektrovorheizer gepumpt und dabei auf 230°C erhitzt. Dann wurde sie von unten nach oben in die Rohrreaktoren eingeleitet, die am Austritt durch ein Druckhalteventil, das auf 150 bar Druck ausgelegt war, verschlossen waren. Die Temperatur betrug in den Rohrreaktoren während der Umsetzung 235 bis 245°C und die mittlere Verweilzeit 4 Stunden.

Das die Reaktionsgefäße verlassende Gemisch wurde in einer Destillierkolonne entspannt. Dabei wurde es in Wasser und ein Tetrahydrofuran-Wasser-Azeotrop zerlegt. Aus dem Sumpf der Destillierkolonne wurde das ursprünglich im Gemisch enthaltene und das bei der Umsetzung entstandene Wasser fortlaufend abgeleitet, während man über Kopf der Kolonne ein bei 66°C siedendes Gemisch aus Tetrahydrofuran und 5,5 Gew.-% Wasser erhielt. Das azeotrope Gemisch wurde auf an sich übliche Weise, z. B. mit Hilfe fester Entwässerungsmittel oder durch extraktive Destillation entwässert. Dabei wurde ein Tetrahydrofuran von sehr hoher Reinheit (über 99,9 Gew.-%) erhalten. Das Tetrahydrofuran hatte eine Carbonylzahl von < 0,01 mg KOH/g und eine Bromzahl von < 0,01 g/100 g. Die Verunreinigung an 2,3- und 2,4-Dihydrofuran betrug < 1 ppm. Sein Gehalt an Butadien-1,3 war < 5 ppm. Die Ausbeute betrug 100 % d. Th. Das erhaltene Tetrahydrofuran ließ sich nach den bekannten Polymerisationsverfahren zu Polytetrahydrofuran mit einer Farbzahl von < 20 APHA polymerisieren. Wird für die gleiche Polymerisation ein technisch hochreines und nicht vorbehandeltes Tetrahydrofuran eingesetzt, so erhält man Polymerisate, die Farbzahlen zwischen 50 und 90 APHA aufweisen.

**Beispiel 2 (Vergleichsbeispiel)**

Bei einer Wiederholung des Beispiels 1 wurde auf die Neutralisation mit Schwefelsäure verzichtet. Dabei wurde das Butandiol nur zu 70 Gew.-% zu Tetrahydrofuran cyclisiert. Dieses ungünstige Ergebnis konnte auch durch eine Verdoppelung der Phosphorsäurekonzentration nicht verbessert werden. Bei dem Versuch, das Ergebnis dieses Vergleichsversuches durch eine Temperatursteigerung auf 280° T zu verbessern, wurde am Reaktionsgefäß eine erheblich höhere Korrosion festgestellt. Die lineare Korrosionsgeschwindigkeit, die beim Beispiel 1 < 0,015 mm/a betrug, wurde in diesem Fall mit 0,5 mm/a ermittelt (Abtrag in mm pro Jahr).

**Beispiel 3**

In der in Beispiel 1 beschriebenen Reaktionskaskade wurde die in Beispiel 1 beschriebene angesäuerte Butandiol-Lösung kontinuierlich durch den Elektrovorheizer gepumpt und dabei auf 245°C erhitzt.
Die Temperatur betrug in den Rohrreaktoren während der Umsetzung 240 bis 255°C. Die mittlere Verweilzeit im Reaktor lag bei 2 Stunden. Das die Reaktionskaskade verlassende flüssige Reaktionsgemisch wurde von unten nach oben durch einen Röhrenwärmetauscher geführt, in dem es bei einer mittleren Verweilzeit von ca. 25 Min. in Abwesenheit einer Gasphase auf 80°C gekühlt wurde. Ein Druckhalteventil, das auf 150 bar ausgelegt war, entspannte das den Kühler verlassende Produkt kontinuierlich in eine Destillierkolonne. Dabei wurde das die Kolonne verlassende Gemisch in Wasser und ein Tetrahydrofuran-Wasser-Azeotrop zerlegt. Aus dem Sumpf der Destillierkolonne wurde das ursprünglich im Gemisch enthaltene und das bei der Umsetzung entstandene Wasser fortlaufend abgeleitet, während man über Kopf der Kolonne ein bei 64°C siedendes Gemisch, das neben ursprünglich vorhandenen Monoalkoholverunreinigungen im Roh-Butandiol im wesentlichen aus Tetrahydrofuran und 5 Gew.-% Wasser bestand. Das azeotrope Gemisch wurde auf an sich übliche Weise, z. B. mit Hilfe fester Entwässerungsmittel oder durch extraktive Destillation entwässert. Die übliche Reindestillation lieferte daraus ein Tetrahydrofuran von sehr hoher Reinheit (über 99,9 Gew.-%). Das Tetrahydrofuran hatte eine Carbonylzahl von (0,01 mg KOH/g und eine Bromzahl von < 0,01 g/100 g. Die Verunreinigung an 2,3- und 2,4-Dihydrofuran betrug < 1 ppm. Sein Gehalt an Butadien-1,3 war < 5 ppm. Die Selektivität betrug 100 % d. Th., die Ausbeute 99,8 %. Das erhaltene Tetrahydrofuran ließ sich nach den bekannten Polymerisationsverfahren zu Polytetrahydrofuran mit einer Farbzahl von < 20 APHA polymerisieren. Wird für die gleiche Polymerisation ein technisch hochreines und nicht vorbehandeltes Tetrahydrofuran eingesetzt, so erhält man Polymerisate, die Farbzahlen zwischen 50 und 90 APHA aufweisen.

**Beispiel 4 (Vergleichsbeispiel)**

Bei einer Wiederholung des Beispiels 2 wurde auf die Neutralisation mit Schwefelsäure verzichtet und der Reaktoraustrag im Verlaufe von 1 bis 5 Min. durch Intensivkühlung auf 40°C abgekühlt. Dabei wurde das Butandiol nur zu 99 Gew.-% zu Tetrahydrofuran cyclisiert. Dieses ungünstige Ergebnis konnte auch durch eine Verdoppelung der Phosphorsäurekenzentration nicht verbessert werden. Bei dem Versuch, das Ergebnis dieses Vergleichsversuches durch eine Temperatursteigerung auf 280°C zu verbessern, wurde am Reaktionsgefäß eine erheblich höhere Korrosion festgestellt. Die lineare Korrosionsgeschwindigkeit, die beim Beispiel 3 > 0,015 mm/a betrug, wurde in diesem Fall mit 0,5 mm/a ermittelt (Abtrag in mm pro Jahr).

**Patentansprüche**

1. Verfahren zur Herstellung von Tetrahydrofuran aus wäßrigen, schwach alkalischen Lösungen von Butandiol-1,4, die durch Umsetzung von Acetylen mit wäßrigem Formaldehyd und katalytische Hydrierung der so hergestellten Butin-2-diol-1,4-Lösung erhalten werden, durch Wasserabspaltung in flüssiger Phase bei höherer Temperatur unter Druck und in Gegenwart einer Säure, dadurch gekennzeichnet, daß man zu der rohen wäßrigen Lösung soviel Schwefelsäure gibt, daß die zur Neutralisation der in der Lösung vorhandenen Basenäquivalente erforderlichen Schwefelsäureäquivalente zumindest erreicht oder um bis zu 20 % überschritten werden, die dabei erhaltene Lösung durch Zugabe von Phosphorsäure ansäuert und dann unter Druck auf Temperaturen von 200 bis 260°C erhitzt.
2. Verfahren nach Anspruch 1, dadurch gekennzeichnet, daß man die Wasserabspaltung bei Temperaturen von 230 bis 245°C vornimmt.

3. Verfahren nach Anspruch 1, <u>dadurch gekennzeichnet</u>, daß man durch Zugabe von Phosphorsäure einen pH-Wert von 2 bis 3 einstellt.

4. Verfahren nach Anspruch 1, <u>dadurch gekennzeichnet</u>, daß man das auf 200 bis 260°C erhitzte flüssige Reaktionsgemisch bei einer mittleren Verweilzeit von mindestens 10 Minuten und in Abwesenheit einer Gasphase auf Temperaturen unter 100°C abkühlt.

5. Verfahren nach Anspruch 1, <u>dadurch gekenneichnet</u>, daß man das flüssige Reaktionsgemisch bei einer mittleren Verweilzeit von 15 bis 30 Minuten auf Temperaturen unter 100°C abkühlt.

## Claims

1. A process for the preparation of tetrahydrofuran from an aqueous, weakly alkaline solution of butane-1,4-diol obtained by reaction of acetylene with aqueous formaldehyde and catalytic hydrogenation of the resulting but-2-yne-1,4-diol solution, by eliminating water in the liquid phase at elevated temperatures, under superatmospheric pressure and in the presence of an acid, wherein sulfuric acid is added to the crude aqueous solution in such an amount that the number of sulfuric acid equivalents necessary for the neutralization of the base equivalents present in the solution is at least attained or exceeded by up to 20 %, and the resulting solution is acidified by adding phosphoric acid and then heated at from 200 to 260°C under superatmospheric pressure.

2. A process as claimed in claim 1, wherein the elimination of water is carried out at from 230 to 245°C.

3. A process as claimed in claim 1, wherein the pH is brought to 2 - 3 by adding phosphoric acid.

4. A process as claimed in claim 1, wherein the liquid reaction mixture which has been heated at 200 - 260°C is cooled to below 100°C during a mean residence time of not less than 10 minutes and in the absence of a gas phase.

5. A process as claimed in claim 1, wherein the liquid reaction mixture is cooled to below 100°C during a mean residence time of from 15 to 30 minutes.

## Revendications

1. Procéde de préparation du tétrahydrofuranne à partir de solutions aqueuses faiblement alcalines de butanediol-1,4 qui ont été obtenues par réaction d'acetylène avec du formaldéhyde aqueux et hydrogénation catalytique de la solution de butyne-2-diol-1,4 ainsi obtenue, par déshydratation en phase liquide à températures élevées sous pression et en présence d'un acide, caractérisé en ce qu'on ajoute de l'acide sulfurique à la solution aqueuse brute de façon au moins à atteindre ou à dépasser jusqu'à 20 % l'équivalent d'acide sulfurique nécessaire pour la neutralisation de l'équivalent de base existant dans la solution, la solution ainsi obtenue est acidifiée par addition d'acide phosphorique puis chauffée sous pression à des températures de 200 à 260°C.

2. Procédé selon la revendication 1, caractérisé en ce qu'on effectue la déshydratation à des températures de 230 à 245°C.

3. Procédé selon la revendication 1, caractérisé en ce que l'on ajuste le pH à une valeur de 2 à 3 par addition d'acide phosphorique.

4. Procédé selon la revendication 1, caractérisé en ce qu'on refroidit le mélange réactionnel liquide chauffé à 200 - 260°C, à des températures inférieures à 100°C en un temps de séjour moyen d'au moins 10 minutes et en l'absence de phase gazeuse.

5. Procédé selon la revendication 1, caractérisé en ce qu'on refroidit le mélange réactionnel liquide à des températures inférieures a 100°C en un temps de séjour de 15 à 30 minutes.